# EUROPEAN PATENT APPLICATION

(11) **EP 4 053 265 A1**
(43) Date of publication of application: **07.09.2022**
(21) Application number: 21160696.7
(22) Date of filing: 04.03.2021
(51) Int. Cl.: C12N 5/077, C12N 15/09, C12N 15/11, C12N 15/63, C07K 14/435, C07K 14/78

(54) **METHOD FOR GENE REPAIR IN PRIMARY HUMAN MUSCLE STEM CELLS (SATELLITE CELLS) IN VITRO AND GENETICALLY REPAIRED HUMAN MUSCLE STEM CELL**

(71) Applicant: Charité - Universitätsmedizin Berlin, 10117 Berlin (DE); Max-Delbrück-Centrum für Molekulare Medizin, 13125 Berlin (DE)
(72) Inventor: Prof. Dr. SPULER, Simone, 14050 Berlin (DE); Dr. ESCOBAR, Helena, 10439 Berlin (DE)
(74) Representative: Maikowski & Ninnemann Patentanwälte Partnerschaft mbB

(57) **Abstract**

The invention relates to a method for gene repair in primary human muscle stem cells (satellite cells) in vitro comprising the following steps: providing a sample of an isolated muscle-fiber containing tissue sample collected from at least one patient with a monogenic muscle disease, wherein the monogenic muscle disease is caused by at least one mutation in at least one gene encoding for at least one muscle protein; isolating and cultivating primary stem cells from said muscle-fiber containing tissue sample, and correcting the at least one mutation in the at least one gene encoding for at least one muscle protein in the cultivated primary stem cells by targeted modification of the at least one mutation by gene editing using CRISPR/Cas-based tools. The invention relates also to genetically repaired human muscle stem cell obtained in such a method.

## Description

The present invention relates to a method for gene repair in primary human muscle stem cells (satellite cells) in vitro and genetically repaired human muscle stem cell obtained by using said method.

### Description

Muscle fibers are syncytial structures with postmitotic nuclei formed by the fusion of myogenic progenitor cells, called myoblasts, during prenatal and postnatal development. Skeletal muscle can regenerate from muscle stem cells (MuSC), also called satellite cells, a pool of tissuespecific stem cells located between the muscle fiber membrane (sarcolemma) and the basal lamina that surrounds every fiber (Mauro A. SATELLITE CELL OF SKELETAL MUSCLE FIBERS. J Biophys Biochem Cytol. 1961;9(2):493-495). In healthy muscle, satellite cells are quiescent or slow cycling. When activated in response to severe damage, they extensively proliferate and give rise to large numbers of myoblasts that fuse to damaged myofibers or to one another to generate new myofibers (Yin H, Price F, Rudnicki MA. Satellite Cells and the Muscle Stem Cell Niche. Physiol Rev. 2013;93(1):23-67). Skeletal muscle regeneration cannot occur without satellite cells. Patients with muscular dystrophy (MD) suffer constant tissue degeneration, which prompts satellite cells to be constantly activated, leading to satellite cell exhaustion, regenerative deficit, and replacement of muscle by fat and connective tissue (Blau HM, Webster C, Pavlath GK. Defective myoblasts identified in Duchenne muscular dystrophy. Proc Natl Acad Sci USA. 1983;80(15):4856-4860).

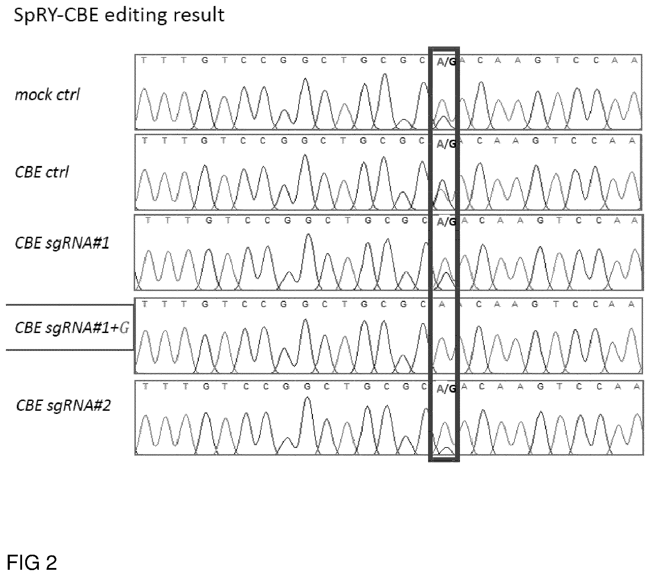

Cell replacement therapies with well-defined and highly myogenic cell populations could represent a safe and long-term treatment avenue for MD patients, however MuSC are scarce and traditionally difficult to manipulate *ex vivo.* In addition, skeletal muscle is the most abundant tissue in the body, thus developing cell replacement therapies for MD patients poses substantial challenges. It has been previously shown that MuSC can be isolated and substantially expanded from human muscle biopsy specimens, and that they maintain *in vivo* regenerative capacity in xenograft models (Marg A, et al. Human satellite cells have regenerative capacity and are genetically manipulable. J Clin Invest. 2014;124(10):4257-4265). Using them in an autologous setting for MD patients would require correcting the genetic defect before reimplantation. A genetically corrected muscle stem cell would be very similar to a healthy ("wild-type") muscle stem cells, but not identical. Formally, this has been demonstrated in base-edited human fibroblasts carrying a mutation in *LMNA* (Koblan et al., In vivo base editing rescues Hutchinson-Gilford progeria in mice. Nature 2021; doi.org/10.1038/S41586-020-03086-7, Fig. 2d).

Muscular dystrophies (MD) are >40 monogenic diseases leading to severe muscular debility. No curative treatment is at present available. Skeletal muscle can regenerate from muscle stem cells and their myogenic precursor cell progeny, myoblasts.

For MD, there is presently no treatment or cure. However, there are ample activities to change this. Companies and research groups are intensively working on solutions. Exon skipping strategies, gene replacement approaches using cDNAs packaged in an AAV vector are in clinical trial stage for some MD. Cell-based therapies for MD are realistic and probably soon available (Bessetti 2020 J. Clin. Invest.). Cell-based therapy approaches can either rely on a "universal donor cell" which lacks individual immunological markers or autologous cells that are "genetically repaired" and transplanted back into the patient.

*In vivo* gene supplementation therapy using adeno-associated viral vectors (AAV) may be suitable for genes up to a certain size. However, the exogenously provided coding sequence is not physiologically regulated in terms of splicing or spaciotemporal expression and poses a risk of insertional mutagenesis. AAV-mediated CRISPR/Cas9 delivery directly into the muscle has enabled highly efficient *in vivo* gene editing in Duchenne's muscular dystrophy (DMD) mice and large animal models and could potentially reach and permanently repair a very large fraction of myonuclei. *In vivo* adenine base editing (ABE) in muscle through AAV delivery has been achieved in a DMD mouse model. However, if not done before substantial degeneration, fatty-fibrous replacement and muscle stem cell exhaustion have occurred, the disease course may not be reversible by either treatment.

In addition, many people have pre-existing antibodies against AAV, Cas9 or other Cas proteins, which interfere with *in vivo* delivery of either of them. Even in patients with no pre-existing immunity, repeated administrations of AAV (for gene supplementation therapy) or AAV-Cas9 (for in vivo gene editing) would not be possible due to adaptive immunity against the virus or the bacterial Cas9 protein triggered by the first administration. This may be especially critical for gene supplementation therapy with AAV vectors, since skeletal muscle tissue has a constant turnover (which is much faster in dystrophic muscle) and thus long-term transgene expression would be hindered by the gradual loss of AAV genomes. Toxicity of systemic AAV administration has also resulted in the death of three patients in a clinical trial of myotubular myopathy due to liver-related adverse events. In two other trials for spinal muscular atrophy (SMA) and DMD, adverse events resulted from an immune response to AAV.

Exon skipping with antisense oligonucleotides is potentially safe but can only be used to treat a subset of patients with a defined type of mutations in genes like *DMD,* where removing one or several exons can be of therapeutic benefit, and needs periodic administration.

Protocols to differentiate induced pluripotent stem cells (iPSC) into cells with myogenic potential in xenograft models have been developed. However, iPSC-derived cells so far lack purity and maturity, and thus pose a risk of tumorigenicity due to the inherent proliferative and pluripotent nature of the parental iPSC. Last, skeletal muscle regeneration cannot occur without muscle stem cells; therefore, the therapeutic potential of other somatic cell types is limited by their non-muscle identity as well as by the requirement of healthy muscle stem cells for proper muscle regeneration.

Non-targeted insertion of therapeutic transgenes via e.g. transposon or integrating viral vectors poses risks of insertional mutagenesis due to uncontrolled insertion sites and adds non-endogenous sequences flanking the transgene, which are needed for the genomic integration process.

Thus, there is a need for an alternative approach for genetic repair of muscle stem cells without having the disadvantages described above.

This object is solved by a method having the features of claim 1.

Accordingly, an *in vitro* method for gene repair in primary human muscle stem cells (satellite cells) is provided, wherein the method comprises the following steps:
- providing a sample of an isolated muscle-fiber containing tissue specimen collected from at least one patient with a monogenic muscle disease , wherein the monogenic muscle disease is caused by at least one mutation in at least one gene encoding for at least one muscle protein;
- isolating and cultivating primary stem cells from said muscle-fiber containing tissue sample, and
- correcting the at least one mutation in the at least one gene encoding for at least one muscle protein in the cultivated primary stem cells by targeted modification of the at least one mutation by gene editing using CRISPR/Cas-based tools.

The present targeted therapeutic method by means of CRISPR/Cas-based, in particular CRISPR/Cas9-based gene/base editing techniques uses primary human myoblasts with regeneration potential which are isolated and collected according to a previously described method (WO 2016/030371 A1). Primary human myoblasts collected by this method are >98% pure and therefore accessible for gene editing. Thus, the present method is able to provide gene edited primary human myoblasts with regenerative capacity.

The transplanted autologous myoblasts in which a phenotypic defect has been therapeutically addressed by gene editing techniques in a site-specific manner can build new muscle tissue in which the pathological phenotype is rescued totally or partially. Furthermore, these cells are able to repopulate the recipient muscle with muscle stem cells that would sustain muscle homeostasis and regeneration long-term.

The present method has several advantages: The cells can be applied in an autologous setting, in which they are unlikely to trigger immune rejection or adverse immune reactions. The edited cells maintain their ability to produce new muscle fibers and repopulate the muscle stem cell pool *in vivo,* thus it is probable that a single administration suffices for a long-term, sustained therapeutic effect. Skeletal muscle tissue has a very low propensity to develop tumors, and so do muscle stem cells and myoblasts. The edited cells can be thoroughly checked for off-target events and biosafety profile prior to reimplantation. These cells are therefore transplantable in an autologous setting with a small and calculable risk.

In an embodiment of the present invention the monogenic muscle disease comprises one of the following: muscular dystrophy (MD) including all types of limb-girdle muscular dystrophy (LGMD), in particular of type LGMD1/D, LGMD2/R (Straub et al., 2018; https://doi.org/10.1016/j.nmd.2018.05.007), all X-linked muscular dystrophies (Emery-Dreyfuss MD, Duchenne MD, Becker MD), and all MDs caused by repeat expansion (i.e. myotonic dystrophy type 1 and type 2) or repeat deletion (facioscapulohumeral muscular dystrophy) mutations. Pax7 myopathy, a very rare disease (Marg et al., Nat Commun. 2019 Dec 18;10(1):5776. doi: 10.1038/s41467-019-13650-z), and VCP myopathy, are included as well.

It is to be understood that the at least one gene mutation in the at least one gene encoding for one muscle protein can be a deletion, insertion, point mutation, repeat expansion, or repeat deletion, in particular a deletion or point mutation.

As mentioned above, precise and efficient gene repair in primary somatic stem and progenitor cells *ex vivo* is increasingly plausible due to the rapid development of CRISPR/Cas-based tools for gene editing, some of which are independent from the cellular DNA repair pathway choice.

Said CRISPR/Cas-based tools, in particular CRISPR/Cas9-based tools, may be used for at least one of the following gene editing approaches: base editing, in particular adenine base editing (ABE), cytidine base editing (CBE), C-to-G base editing (CGBE), glycosylase base editing (GBE), prime editing, non-homologous end joining (NHEJ), microhomology-mediated end joining (MMEJ) and/or homology-directed repair (HDR). In general, at least one transport system in form of a vehicle, carrier or molecule (in particular, DNA, RNA, protein or viral vector) that is or carries the information for the synthesis of the components required for CRISPR/Cas-based genetic modifications is introduced into at least one primary human stem cell carrying the genetic defect.

The different modes of delivery comprise:
- For the Cas enzyme/base editor/prime editor: DNA (e.g. plasmid DNA, minicircle DNA), mRNA, protein, viral vector;
- For the guide RNA: DNA (e.g. plasmid DNA, minicircle DNA), RNA, viral vector;
- For the homology template (in case of HDR): DNA (can be single stranded like an ssODN or double stranded like a plasmid of minicircle), viral vector (e.g. AAV)

Non-integrating viral vectors are preferred.

Base editors are RNA-programmable deaminases that enable precise single-base conversions in DNA or RNA. Base editors do not create double-stranded DNA breaks and therefore minimize the formation of undesired editing byproducts, including insertions, deletions, translocations, and other large-scale chromosomal rearrangements.

Adenine base editing (ABE) enables the precise targeted conversion of adenine into guanine nucleotides without inducing DNA double-strand breaks (Gaudelli NM, et al. Programmable base editing of A•T to G•C in genomic DNA without DNA cleavage. Nature. 2017;551(7681):464-471). An ABE consists of a catalytically impaired Cas9 in fusion with an adenine deaminase enzyme (TadA) that converts adenine into inosine on the single stranded DNA bubble created by Cas9 binding to a target site. Inosine is subsequently replaced by guanine. To be accessible to the deaminase, the target adenine must be located at a defined distance from the protospacer adjacent motif (PAM), the so-called ABE activity window. Because of its predictable outcome, high precision and reduced off-target effects (20, 21), ABE is a safe gene editing tool.

Cytosine base editors (CBEs) enable efficient, programmable reversion of T•A to C•G point mutations in the human genome. Cytosine base editors (CBEs) are comprised of a cytidine deaminase fused to an impaired form of Cas9 (D10A nickase) tethered to one (BE3) or two (BE4) monomers of uracil glycosylase inhibitor (UGI). This architecture of CBEs enables the conversion of C•G base pairs to T•A base pair in human genomic DNA, through the formation of a uracil intermediate.

More recently, base editors have been developed (Pin-point^{™}) in which the effector domain (deaminase) is not fused to Cas9. Instead, the effector is fused to a cognate ligand for an RNA aptamer that is included in the gRNA, thereby facilitating the recruitment of the effector to the Cas9/gRNA complex (Collantes et al., 2021, CRISPR J, DOI: 10.1089/crispr.2020.0035)

Non-homologous end joining (NHEJ) repair double-strand breaks (DSB) in DNA (for example, generated by Cas9 cutting) often introduces small insertions and/or deletions (indels) at the repair site.

Homology directed repair (HDR) pathway makes use of a provided DNA template with regions of homology to the target site, hence allowing for the generation of desired alterations.

In a more specific embodiment, the at least one mutation is located in at least one of the following genes: *LMNA* encoding for lamin A/C, *CAPN3* encoding for calpain 3, *DYSF* encoding for dysferlin, *SGCA* encoding for α-sarcoglycan, *VCP* encoding for valosin containing protein, *PAX7* encoding for paired box 7, *NCAM1* encoding for neural cell adhesion molecule *1, DMD* encoding for dystrophin.

α-Sarcoglycan is a 50 kDa transmembrane protein, part of the sarcoglycan complex and the dystrophin-associated protein complex (DAPC). The DAPC protects muscle fibers from mechanical stress and its dysfunction leads to various forms of muscular dystrophy (MD).

Loss-of-function mutations in *SGCA,* encoding α-sarcoglycan, cause limb-girdle muscular dystrophy 2D / 3R, an early onset, severe and rapidly progressive form of muscular dystrophy affecting equally girls and boys. Patients suffer from muscle degeneration and atrophy affecting the limbs, respiratory muscles, and the heart.

*SGCA* has 9 coding exons and is expressed in striated muscle (2, 3). Disease-causing mutations are spread along the entire length of the gene without defined mutational hotspots (4, 5). However, some mutations like c.157G>A have been reported more frequently (Fendri K, Kefi M, Hentati F, Amouri R. Genetic heterogeneity within a consanguineous family involving the LGMD 2D and the LGMD 2C genes. Neuromuscul Disord. 2006;16(5):316-320).

In an embodiment *SGCA* mutations, in particular c.157G>A mutation, is reversed or repaired by adenine base editing (ABE).

Classical laminopathy refers to diseases caused by mutations in the *LMNA* gene, coding for the nuclear lamina protein lamin A/C.

In an embodiment *LMNA* mutations, in particular the c.1366A>G mutation, is reversed or repaired by cytosine based editing (CBE).

Mutations in the *DYSF* gene, encoding for dysferlin, cause weaknesses mainly of posterior hip and near hip thigh muscles, calf pseudohypertrophy and shoulder muscles. One mutation in *DYSF* is based on a deletion of G on position 4782 of the *DYSF* coding sequence, which induces a frameshift and a premature stop codon. Another mutation G>C is four positions downstream.

In an embodiment *DYSF* mutations, in particular G deletion, are reversed or repaired by CRISPR/Cas9-induced non-homologous end joining (NHEJ). Specifically, a +1A insertion restores the *DYSF* reading frame resulting in a removal of the premature stop codon. Calpain 3, the protein encoded by *CAPN3,* is a cysteine-protease predominantly expressed in skeletal muscle. Mutations in *CAPN3* cause LGMD2A, a progressive skeletal muscle disorder without treatment and the most common form of LGMD worldwide. Deletion *CAPN3* c.550deIA causes a frameshift in exon 4, which creates a premature stop codon.

In an embodiment *CAPN3* mutations, in particular A deletion, are reversed or repaired by CRISPR/Cas9-induced non-homologous end joining (NHEJ). Specifically, a +1A insertion restores the reading frame resulting in a removal of the premature stop codon. The +1 insertion in exon 4 of *CAPN3* to reverse *CAPN3* c.550deIA is exempted if the repair is plasmid based. All other approaches such as mRNA-based repair approaches are included.

As mentioned, the method for providing a sample of an isolated muscle-fiber containing tissue sample collected from at least one patient with muscular dystrophy has been previously described (WO 2016030371 A1).

Specifically, the primary stem cells from said muscle-fiber containing tissue sample collected from a patient with muscular dystrophy are cultivated by a treatment without oxygenation under hypothermic conditions having a defined temperature and a defined atmosphere, wherein the temperature does not exceed 15 °C and the atmosphere has an oxygen content not exceeding 21 % (v/v), for a time of 4 days to 4 weeks.

The cultivation under these conditions leads to an enrichment of stem cells in the sample such that approximately 70 to 100 % of all viable cells in the sample are cultivated stem or derivatives from cultivated stem cells after a first period of time. The cultivation takes place by using a medium that is suited or adapted for the stem cells to be cultivated.

In an embodiment, the temperature does not exceed 14 °C, in particular 13 °C, in particular 12 °C, in particular 11 °C, in particular 10 °C, in particular 9 °C, in particular 8 °C, in particular 7 °C, in particular 6 °C, in particular 5 °C, in particular 4 °C, in particular 3 °C, in particular 2 °C in particular 1 °C, in particular 0 °C. In an embodiment, the temperature is in a range of 0 °C to 15 °C, in particular 1 °C to 14 °C, in particular 2 °C to 13 °C, in particular 3 °C to 12 °C, in particular 4 °C to 11 °C, in particular 5 °C to 10 °C, in particular 6 °C to 9 °C, in particular 7 °C to 8 °C.

In an embodiment, the atmosphere has an oxygen content not exceeding 20 volume %, in particular 19 % (v/v), in particular 18 % (v/v), in particular 17 % (v/v), in particular 16 % (v/v), in particular 15 % (v/v), in particular 14 % (v/v), in particular 13 % (v/v), in particular 12 % (v/v), in particular 11 % (v/v), in particular 10 % (v/v), in particular 9 % (v/v), in particular 8 % (v/v), in particular 7 % (v/v), in particular 6 % (v/v), in particular 5 % (v/v), in particular 4 % (v/v), in particular 3 % (v/v), in particular 2 % (v/v), in particular 1 % (v/v), in particular not exceeding any of the before-mentioned oxygen contents. Conditions having an atmosphere with an oxygen content of less than 20 % (v/v) are often also referred to as hypoxic conditions.

In an embodiment, the atmosphere has an oxygen content lying in a range of 1 % (v/v) to 21 % (v/v), in particular of 2 % (v/v) to 20 % (v/v), in particular of 3 % (v/v) to 19 % (v/v), in particular of 4 % (v/v) to 18 % (v/v), in particular of 5 % (v/v) to 17 % (v/v), in particular of 6 % (v/v) to 16 % (v/v), in particular of 7 % (v/v) to 15 % (v/v), in particular of 8 % (v/v) to 14 % (v/v), in particular of 9 % (v/v) to 13 % (v/v), in particular of 10 % (v/v) to 12 % (v/v), in particular of 3 % (v/v) to 11 % (v/v).

In an alternative embodiment, the atmosphere has an oxygen content not exceeding 30 volume %, in particular not exceeding 29 % (v/v), in particular not exceeding 28 % (v/v), in particular not exceeding 27 % (v/v), in particular not exceeding 26 % (v/v), in particular not exceeding 25 % (v/v), in particular not exceeding 24 % (v/v), in particular not exceeding 23 % (v/v), in particular not exceeding 22 % (v/v), in particular not exceeding 21 % (v/v),

In an embodiment, the temperature is in a range of 0 °C to 10 °C and the oxygen content is in a range of 0 % (v/v) to 8 % (v/v). In an embodiment, the temperature is in a range of 2 °C to 5 °C and the oxygen content is in a range of 2 % (v/v) to 5 % (v/v). In an embodiment, the temperature is in a range of 3 °C to 4 °C and the oxygen content is in a range of 3 % (v/v) to 4 % (v/v). In an embodiment, the temperature does not exceed 10 °C and the oxygen content does not exceed 8 % (v/v).

In an embodiment, growth factors are added to the medium in which the stem cells are cultivated. In an embodiment, growth factors are only added if the stem cells are cultivated for more than 2 weeks, in particular for more than 2 weeks at 4 °C. In particular in case of HMFF as sample and satellite cells to be cultivated, a medium with low serum content is suited. A well-suited medium is a serum-reduced optimized minimal essential medium, such as OptiMEM, obtainable from Life Technologies. It turned out that a duration of the first period of time of 5 days to 2 weeks, in particular of 6 days to 1 week, in particular 1 week is particularly suited for stem cell cultivation and enrichment.

In an embodiment, the sample is an isolated tissue sample. It can be isolated from a patient by standard methods, such as a biopsy. In an embodiment, the sample is an isolated muscle fiber fragment. Human muscle fiber fragments (HMFFs) are particularly suited and easily obtainable from a muscle biopsy specimen.

In an embodiment, the stem cells are cultivated in a united cell structure. This can be achieved best by a supportive structure that enables a united cell structure. In an embodiment, this supportive structure is the natural structure in which the cells grow in a body (such as a HMFF which is very well suited in the context of this invention to cultivate satellite cells). In another embodiment, this supportive structure is an artificial structure mimicking or closely resembling the natural structure in which the cells grow in a body.

After a sufficient amount of primary stem cells are cultivated the above described gene editing methods using CRISPR/Cas-based tools are applied to the primary stem cells in order to correct the mutations in the muscle proteins.

Following gene repair, the human muscle cells are screened for the desired gene repair/editing event. A selection step, for example by FACS, may be performed. In particular, a fluorescent molecule expressed by transfected/edited cells may be used, or selection may be based on live cell staining of extracellular epitopes.

The genetically modified primary stem cells are further cultivated.

In one embodiment the modified primary stem cells are fused into multinucleated myotubes in vitro.

In a further aspect, the invention also relates to genetically repaired or modified human muscle stem cells obtained after carrying out above method.

In an embodiment the genetically repaired human muscle stem cells comprise at least one gene encoding for at least one muscle protein, wherein the at least one gene underwent a targeted modification of at least one mutation in said gene, for example by base editing or prime editing using CRISPR/Cas-based tools as described in detail above.

As previously indicated, a genetically corrected muscle stem cell obtained by the present method differs from a healthy ("wild-type") muscle stem cells, and is thus not identical to the wild-type. This has been demonstrated in base-edited human fibroblasts carrying a mutation in *LMNA* (Koblan et al., In vivo base editing rescues Hutchinson-Gilford progeria in mice. Nature 2021; doi.org/10.1038/s41586-020-03086-7, Fig. 2d).

In a preferred embodiment, the genetically repaired human muscle stem cells comprise at least one modified gene encoding for at least one of the following muscle proteins: *LMNA* encoding for lamin A/C, *CAPN3* encoding for calpain 3, *DYSF* encoding for dysferlin, *SGCA* encoding for α-sarcoglycan, *VCP* encoding for valosin containing protein, *PAX7* encoding for paired box 7, *NCAM1* encoding for neural cell adhesion molecule 1, *DMD* encoding for dystrophin.

In a further aspect, the invention also relates to the medical use of genetically repaired or modified muscle stem cells. Thereby, the stem cells are intended to be used in cell replacement therapies for muscular dystrophy (MD), including all types of limb-girdle muscular dystrophy (LGMD), in particular of type LGMD1/D, LGMD2/R (Straub et al., 2018; https://doi.org/10.1016/j.nmd.2018.05.007), all X-linked muscular dystrophies, in particular Emery-Dreyfuss MD, Duchenne MD, Becker MD, and all MDs caused by repeat expansion (i.e. myotonic dystrophy type 1 and type 2) or repeat deletion (facioscapulohumeral muscular dystrophy) mutations. The stem cells can be also used for treatment of Pax7 myopathy (Marg et al., Nat Commun. 2019 Dec 18;10(1):5776. doi: 10.1038/s41467-019-13650-z) or VCP myopathy.

Herewith, a method of transplanting cultivated stem cells to a subject or patient in need thereof is disclosed, the method comprising administering genetically repaired or modified stem cells cultivated as outlined above to the patient, in particular by autologous transplantation.

Herewith, a method of treating a patient suffering from a muscular dystrophy is disclosed, the method comprising administering genetically modified muscular stem cells to the patient. In an embodiment, the muscular stem cells are satellite cells. In an embodiment, the genetically modified stem cells are administered as cell suspensions. Delivery in form of a tissue supportive structure system is also feasible.

The invention will be explained in more detail in the following with respect to exemplary embodiments and Figures. It shows:
- Fig. 1A-E: ABE repairs the *SGCA* c.157G>A mutation in patient and carrier primary muscle stem cells without detectable off-target editing;
- Fig. 2: CBE repairs the *LMNA* c.1366 A>G mutation in patient-derived cells;
- Fig. 3: *DYSF* editing in patient primary MuSC/myoblasts.

### Example 1: Gene editing on SGCA related muscular dystrophy patient-derived cells

### a) Isolation of primary MuSC from a patient and a carrier with a compound heterozygous SGCA c.157G>A mutation.

Primary MuSC from muscle biopsy specimens obtained from a 10-year old male LGMD2D patient carrying a compound heterozygous *SGCA* c.157G>A mutation and from a related carrier were isolated and characterized.

*Primary MuSC isolation and culture.* Immediately after the biopsy procedure, the muscle specimen was transferred into Solution A for transport (30 mM HEPES, 130 mM NaCl, 3 mM KCI, 10 mM D-glucose and 3.2 µM Phenol red, pH 7.6). The fresh muscle specimen was manually dissected, and fragments were subjected to hypothermic treatment at 4-6°C for 2 to 7 days prior to downstream processing for MuSC isolation. Oligoclonal MuSC colonies were obtained following mechanical dissection as described (Marg A, et al. Human muscle-derived CLEC14A-positive cells regenerate muscle independent of PAX7. Nat Commun. 2019;10(1):5776). The outgrowing colonies were expanded until passage 4 and characterized prior to cryopreservation. To enhance the probability of available MuSC in difficult-to-handle biopsy specimens, classical purification was performed in parallel (Blau HM, Webster C, Pavlath GK. Defective myoblasts identified in Duchenne muscular dystrophy. Proc Natl Acad Sci USA. 1983;80(15):4856-4860). All cell populations used in this study were ≥95% positive for Desmin. To induce myoblast-to-myotube fusion, medium was switched to Opti-MEM I Reduced Serum Media (Thermo Fisher Scientific) once cells reached confluence.

Primary MuSC cultures from patient and carrier were 95-100% Desmin+ and expressed the myogenic markers Pax7, MyoD, Myf5 and the proliferation marker Ki-67. The c.157G>A mutation affects the last coding nucleotide of exon 2.

### b) ABE results in >90% correction of SGCA c.157G>A in primary human MuSC without detectable off-target editing

It was found that c.157G>A is an ideal ABE target, as it is located 15 bp upstream of an -NGG PAM (equivalent to protospacer position 6, thus in the center of the ABE activity window). No other adenines are located within the ABE activity window, so undesired bystander edits are unlikely. It was first assessed if ABE can be used to repair the c.157G>A mutation in patient iPSC.

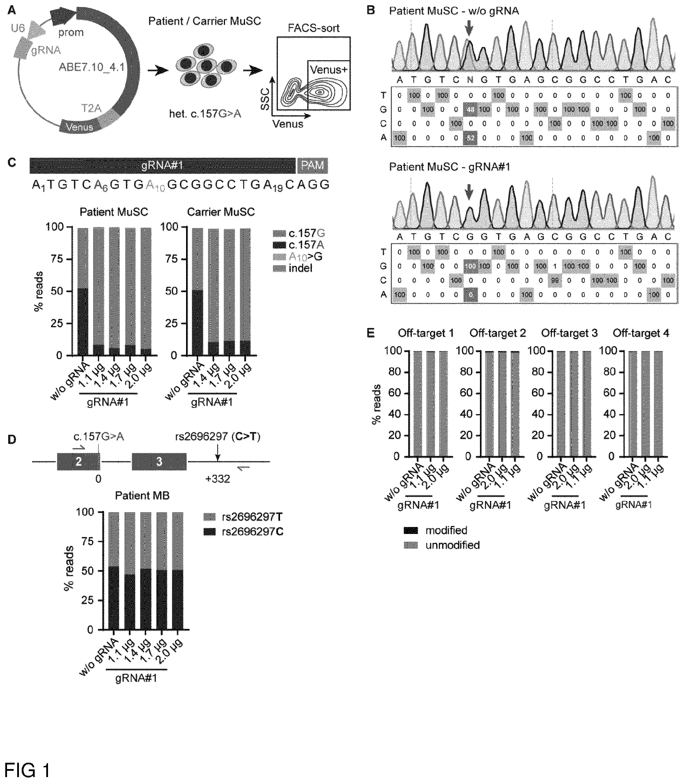

The MuSC from the patient were transfected with various amounts of a plasmid encoding ABE7.10_4.1 (Figure 1A) a vector based on ABE7.10 (Gaudelli NM, et al. Programmable base editing of A•T to G•C in genomic DNA without DNA cleavage. Nature. 2017;551 (7681):464-471) containing a codon-optimized Cas9(D10A) nickase N-terminally fused to the TadA heterodimer, followed by a T2A-Venus cassette under control of the CAG promoter, and a U6 promoter-driven sgRNA expression cassette.

It was enriched for Venus-positive cells (Figure 1A) via FACS and ABE efficiency was assessed using EditR (Kluesner MG, et al. EditR: A Method to Quantify Base Editing from Sanger Sequencing. CRISPR J. 2018;1(3):239-250).

*Human primary MuSC transfection and sorting.* Human primary MuSC were plated one day before transfection at a density of 55,000 cells/9.5 cm² in Skeletal Muscle Cell Growth Medium (SMCGM, Provitro) and transfected using Lipofectamine^{®}3000 (Thermo Fisher Scientific) following manufacturer's instructions. SMCGM was exchanged after one day. Two days after transfection, cells were collected for FACS-sorting in PBS containing 50% SMCGM, 0.05 mM EDTA and 100 µg/ml Primocin^{™}. Venus-positive cells were sorted using a FACSAria Fusion cell sorter (BD Biosciences) and cultured in SMCGM. 100 µg/ml Primocin^{™} were added to the culture medium for two days.

All vector concentrations resulted in >99% c.157G nucleotide rates in patient and carrier MuSC as analyzed by EditR (Figure 1B). Then amplicon sequencing was performed with subsequent analysis by Crispresso2 (26) and confirmed high c.157G nucleotide rates of >90% for patient MuSC and >85% for carrier MuSC (Figure 1C). Bystander A>G editing at protospacer position 10 was detected in a very low (0.2-2%) percentage of reads. In two samples 1.1% and 0.3% of reads containing indels were detected. Omission of gRNA did not result in either A>G editing or indels (Figure 1C).

An equal representation of both alleles in the amplicon sequencing data was confirmed, thus ruling out detection bias (Figure 1D).

No Cas9-dependent off-target editing events were detected at the top predicted (by CRISPOR) off-target loci containing A nucleotides in the ABE activity window with either the lowest or highest vector concentration (Figure 1E).

It was found that ABE7.10_4.1 induced efficient c.157A>G conversion when combined with a suitable gRNA (gRNA#1), with only minimal (0.2-2%) bystander A>G edits detected by amplicon sequencing and Crispresso 2 analysis.

It was thus concluded that the *SGCA* c.157G>A mutation can be repaired in human primary MuSC with very high efficiency and specificity via ABE. Repaired *SGCA* c.157G>A is hereafter referred to as *SGCA* c.157Grep.

### c) SGCA c.157Grep primary MuSC show normal α-sarcoglycan mRNA and protein expression.

To assess the functional outcome of ABE, α-sarcoglycan mRNA and protein expression in *SGCA* c.157Grep myotubes was analyzed. It was found that the splicing defect was rescued as shown by the increase in α-sarcoglycan transcripts containing exon 2 in *SGCA* c.157Grep compared to unedited patient and carrier myotubes, reaching levels similar to control 3 (het. c.748-2A>G carrier) in the case of patient myotubes. Furthermore, total *SGCA* mRNA levels increased in patient myotubes following ABE, probably because co-skipping of exons 2+3 (but not exon 2 alone) induces a frameshift leading to a premature stop codon and thus likely nonsense mediated mRNA decay (NMD). Western blot and immunostaining analysis revealed that α-sarcoglycan protein was restored in *SGCA* c.157Grep patient cells.

### d) SGCA c.157Grep primary patient MuSC are viable, proliferative, and myogenic.

Primary cells are especially susceptible to stress induced by extensive manipulation. Primary MuSC derived from MD patients with mutations in genes responsible for membrane integrity are particularly vulnerable. A decrease in cell proliferation in the first days following transfection and sorting as compared to untransfected patient MuSC was observed. However, Venus-positive cells (≥ 48% of the source cell population) proliferated extensively after sorting and were further expanded for at least 2-3 passages before cryopreservation. *SGCA* c.157Grep primary MuSC could readily fuse into multinucleated myotubes *in vitro.* Moreover, the pattern of α-sarcoglycan localization was indistinguishable from control myotubes.

### e) SGCA c.157Grep primary MuSC regenerate muscle and repopulate the satellite cell niche in vivo.

*SGCA* c.157Grep primary MuSC were transplanted into irradiated anterior tibial muscles of immunocompromised NSG mice. It was found that *SGCA* c.157Grep patient MuSC gave rise to abundant human muscle fibers. Furthermore, the satellite cell niche between the sarcolemma and the basal lamina was populated with numerous Pax7+ cells of human origin. Taken together, *SGCA* c.157Grep patient MuSC are capable of both myofiber regeneration and reconstitution of the satellite cell compartment *in vivo.*

*Human MuSC transplantation. SGCA* c.157Grep patient MuSC that were 99% Desmin+, 27% Pax7+, 25% Ki-67+, 66% MyoD+ and 40% Myf5+ were used for transplantation. 6-week old male NOD.Cg-*Prkdc^{scid}II2rg^{tm1Wjl}*/SzJ (NSG) mice were purchased from Charles River Laboratories 1 week before the experiment. Animal housing and hygienic monitoring followed FELASA recommendations. Focal irradiation of the recipient hind limbs was performed two days prior to cell transplantation as described (17, 18). Two injections of 5.5 µl containing 2,5×10⁴ cells in a sterile PBS + 2% FCS solution were performed following parallel trajectories into the medial portion of the TA muscle (in total 5×10⁴ cells per grafted muscle) as described (18). Mice were sacrificed 19 days after cell transplantation. TA muscles were cryopreserved in liquid nitrogen-chilled isopentane, mounted in gum tragacanth and stored at -80°C.

### Example 2: Gene editing in LMNA related muscular dystrophy patient-derived cells

Classical laminopathy refers to diseases caused by mutations in gene *LMNA,* coding for nuclear lamina protein lamin A/C. The state-of-the-art gene editing tools provide the possibility to correct the mutations at the genomic level, especially the powerful base editors in correcting single nucleotide mutations without DNA double strand breaks.

An 8-year old girl was diagnosed with muscular dystrophy, carrying a dominant mutation in *LMNA* c.1366 A>G. Base editing was performed at first with patient derived induced pluripotent stem cells (iPSC), an unlimited cell source to test the editing efficiencies.

The initial test was done with co-transfection of two vectors to iPSC with transfection reagent Lipofectamine^{®}3000 in mTeSR Plus stem cell medium. One DNA vector carries CBE4max - an enhanced version of the first reported cytidine base editor and SpRY, a new near-PAMless Cas9 able to edit mutations that were previously uneditable by classical Cas9, and the other vector expresses the sgRNA.

Initial editing results showed a conversion of G to A, although the editing efficiency is low due to low transfection efficiency (Fig. 2).

Based on the preliminary results of an ABE editing project, the transfection with synthesized Cas9 mRNA instead of a DNA vector revealed higher editing efficiency. Thus editing efficiency can be improved via transfection with the synthesized mRNA containing the CBE4max and SpRY sequences along with synthesized sgRNA.

Following by the optimized CBE editing in iPSC, patient derived muscle stem cells will be edited with the same protocol and ultimately the corrected muscle stem cells will be used for the transplantation therapy to improve the muscle function of patients.

### Example 3: DYSF gene editing in patient primary MuSC/myoblasts

### a) Human primary myoblast culture

Human primary myoblasts (hPMs) were grown in humidified atmosphere containing 5% CO₂ at 37°C on 10 cm plastic dishes (Corning) in skeletal muscle cell growth medium (SMCGM) (Provitro) enriched with fetal calf serum (FCS) supplement mix (Provitro) and 2.72 mM glutamine (GlutaMAX^{™}, Thermo Fisher Scientific). For cell passaging, hPMs were washed with Dulbecco's phosphate-buffered saline (DPBS) (Thermo Fisher Scientific) and treated with 0,25 % Trypsin/EDTA (Thermo Fisher Scientific) at 37°C for 5 min.

Detached cells were collected in SMCGM + supplement to a dilution of 1:10 and centrifuged at 200 g for 5 min at room temperature (RT). Pellet was resuspended in an appropriate volume of SMCGM + supplement and seeded at a density of 1-2*10⁴ cells/cm² on 10 cm plates. Cells were passaged every 2-3 days according to growth rate/confluence.

### b) CRISPR/Cas9-based gene editing

**Table 1: sgRNA sequences used for the CRISPR/Cas9 experiment.**

| **Locus** | **Target allele** | **Guide ID** | **Guide sequence** | **PAM** | **Orientation** |
|---|---|---|---|---|---|
| *DYSF* | Mutant exon 44 | *DYSF*ex44mut#3 | AAATAGGGGTCCAGCGTGC | GGG | sense |

### c) Lipo-transfection of the Cas9/sgRNA complex

For the CRISPR/Cas9 experiments, hPMs were seeded at a density of 75,000 cells/well of a 6-well plate one day before transfection. 1 µg SpCas9::Venus plasmid DNA (with and without sgRNA) was transfected using Lipofectamine^{®}3000 transfection reagent (Invitrogen, Germany), according to the manufacturer's instructions. 48h after transfection the Venus-positive cells were sorted using FACSAria Fusion (BD). Sorted cells were plated again and expanded for genomic DNA isolation and dysferlin protein analysis via flow cytometry or immunofluorescence staining, respectively.

For dysferlin immunostaining, hPMs were seeded in 8-well ibidi µ-Slides (IBIDI GmbH Martinsried, Cat. # 80826) in SMCGM + supplement and allowed to proliferate until 70%/80% confluence was reached. Myoblast fusion was induced by switching the culture medium to OptiMEM (Thermo Fisher Scientific). After four days, cells were fixed and stained with an antibody against the N-terminal part of Dysferlin (ab124684, Abcam).

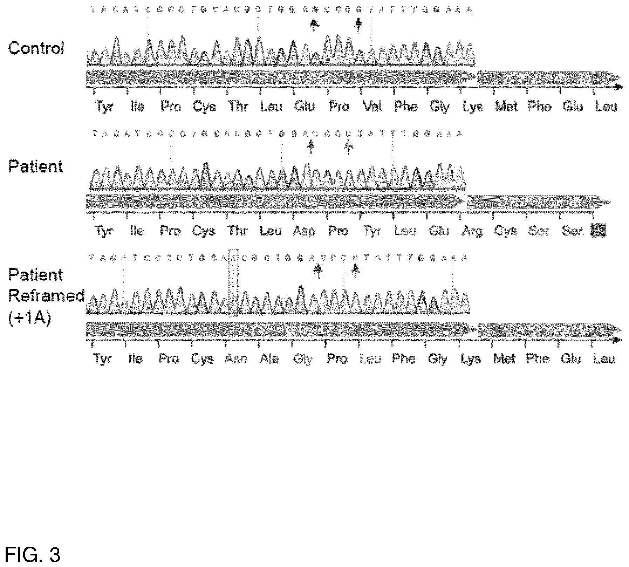

Figure 3 illustrates the results obtained for *DYSF* gene editing. Genomic DNA was isolated from a control (upper panel) and a patient carrying a homozygous *DYSF* c.4782_4786delinsCCC mutation in exon 44 (middle panel). The c.4782_4786delinsCCC mutation is a deletion of a G nucleotide in position c.4782 of the *DYSF* coding sequence (which induces a frameshift) and a G>C substitution four positions downstream.

The +1A insertion restores the *DYSF* reading frame, resulting in a removal of the premature Stop codon, whilst four amino acids (indicated in blue) differ from the wild-type protein sequence (lower panel). Dysferlin protein expression is rescued in both patient iPSC and primary MuSC/hPM after restoring the reading frame by the +1A insertion. Dysferlin localization in +1A re-framed patient myotubes is similar to control myotubes.

### Example 4: CAPN3 gene editing in patient primary myoblasts

Calpain 3, the protein encoded by *CAPN3,* is a cysteine-protease predominantly expressed in skeletal muscle. Mutations in *CAPN3* cause limb-girdle MD Type 2A (LGMD2A), a progressive skeletal muscle disorder without treatment and the most common form of LGMD worldwide.

Human primary muscle stem cells from 35 patients carrying 37 different *CAPN3* mutations were isolated and expanded. 20% of the patients carry the well-known founder mutation *CAPN3* c.550deIA causing a frame shift in exon 4, which creates a premature stop codon. In most cases, patients carry compound heterozygous *CAPN3* c.550deIA mutations, two patients with homozygous c.550deIA mutations are also part of the cohort.

Primary MuSC from a homozygous patient were isolated, expanded and transfected with a plasmid, which carries mutation-specific sgRNAs and SpCas9::Venus. After cell sorting and expansion, a subsequent in-depth sequence analysis of the *CAPN3* c.550 DNA region showed base insertions and deletions (indels) at the targeted *CAPN3* locus with an efficiency of up to 60%. One of the sgRNAs had a preference of a +1 bp insertion at the position of the mutation demonstrating an indel signature bias of specific sgRNAs and reframing of the open reading frame (ORF).

The effects on protein level were analyzed using a custom made monoclonal anti-Calpain 3 antibody suitable for immunostaining.

## Claims

1. Method for gene repair in primary human muscle stem cells (satellite cells) in vitro comprising the following steps
- providing a sample of an isolated muscle-fiber containing tissue sample collected from at least one patient with monogenic muscle disease, wherein the monogenic muscle disease is caused by at least one mutation in at least one gene encoding for at least one muscle protein;
- isolating and cultivating primary stem cells from said muscle-fiber containing tissue sample, and
- correcting the at least one mutation in the at least one gene encoding for at least one muscle protein in the cultivated primary stem cells by targeted modification of the at least one mutation by gene editing using CRISPR/Cas-based tools.

2. Method according to claim 1, **characterized in that** the monogenic muscles disease comprises one of the following: muscular dystrophy including all types of limb-girdle muscular dystrophy (LGMD), in particular of type LGMD1/D, LGMD2/R, all X-linked muscular dystrophies (Emery-Dreyfuss MD, Duchenne MD, Becker MD), all MDs caused by repeat expansion (i.e. myotonic dystrophy type 1 and type 2) or repeat deletion (facioscapulohumeral muscular dystrophy) mutations, Pax7 myopathy or VCP myopathy.

3. Method according to one of the preceding claims, **characterized in that** the at least one gene mutation can be a deletion, insertion or point mutation, repeat expansion, or repeat deletion, in particular a deletion or point mutation.

4. Method according to one of the preceding claims, **characterized in that** the at least one mutation is located in at least one of the following genes: *LMNA* encoding for lamin A/C, *CAPN3* encoding for calpain 3, *DYSF* encoding for dysferlin, *SGCA* encoding for alpha-sarcoglycan, *VCP* encoding for valosin containing protein, *PAX7* encoding for paired box 7, *NCAM1* encoding for neural cell adhesion molecule 1 or *DMD* encoding for dystrophin.

5. Method according to one of the preceding claims, **characterized in that** the gene editing using CRISPR/Cas-based tools comprises at least one of the following: adenine base editing (ABE), cytidine base editing (CBE), C-to-G base editing (CGBE), glycosylase base editing (GBE), prime editing non-homologous end joining (NHEJ), microhomology-mediated end joining (MMEJ) and/or homology-directed repair (HDR).

6. Method according to one of the preceding claims, **characterized in that** the primary stem cells from said muscle-fiber containing tissue sample are cultivated by a treatment without oxygenation under hypothermic conditions having a defined temperature and a defined atmosphere, wherein the temperature does not exceed 15°C and the atmosphere has an oxygen content not exceeding 21 % (v/v), and wherein the first period of time is 4 days to 4 weeks.

7. Method according to claim 6, **characterized in that** the temperature does not exceed 10°C and the oxygen content does not exceed 10 % (v/v).

8. Method according to one of the preceding claims, **characterized in that** genetically modified primary stem cells are further cultivated.

9. Genetically repaired human muscle stem cell obtained after carrying out a method according to one of the preceding claims.

10. Genetically repaired human muscle stem cell, **characterized in that** it comprises at least one gene encoding for at least one muscle protein, wherein the at least one gene underwent a targeted modification of at least one mutation in said gene.

11. Genetically repaired human muscle stem cell according to claim 10, **characterized in that** the at least one modified gene encodes for at least one of the following muscle proteins: *LMNA* encoding for lamin A/C, *CAPN3* encoding for calpain 3, *DYSF* encoding for dysferlin, *SGCA* encoding for alpha-sarcoglycan, *VCP* encoding for valosin containing protein, *PAX7* encoding for paired box 7, *NCAM1* encoding for neural cell adhesion molecule 1 or *DMD* encoding for dystrophin.

12. Genetically repaired human muscle stem cell for use in cell replacement therapies for muscular dystrophy, in particular all types of limb-girdle muscular dystrophy (LGMD), in particular of type LGMD1/D, LGMD2/R, all X-linked muscular dystrophies (Emery-Dreyfuss MD, Duchenne MD, Becker MD), all MDs caused by repeat expansion (i.e. myotonic dystrophy type 1 and type 2) or repeat deletion (facioscapulohumeral muscular dystrophy) mutations, and Pax7 or VCP myopathy.
